# EUROPEAN PATENT APPLICATION

(11) **EP 4 011 420 A1**
(43) Date of publication of application: **15.06.2022**
(21) Application number: 20212679.3
(22) Date of filing: 09.12.2020
(51) Int. Cl.: A61M 5/178, A61B 5/00

(54) **ELECTRICALLY CONDUCTIVE FIXATION AND METHOD FOR FIXATING A PRINTED CIRCUIT BOARD IN A MEDICAL DEVICE**

(71) Applicant: Ypsomed AG, 3401 Burgdorf (CH)
(72) Inventor: Scheurer, Simon, 3006 Bern (CH); Schrul, Christian, 3400 Burgdorf (CH); Gersbach, Jonatan, 3414 Oberburg (CH); Bernhard, Mario, 3400 Burgdorf (CH); Frey, Lukas, 4900 Langenthal (CH); Scheidegger, Thomas, 3400 Burgdorf (CH)
(74) Representative: Meier Obertüfer, Jürg

(57) **Abstract**

A medical device comprising a housing made from a polymeric material and a printed circuit board (PCB) fixated to the housing by means of a mechanical connection. The medical device further comprises a sensor located in or forming part of the housing and an electrical contact located between the housing and the printed circuit board and formed between the sensor and the printed circuit board. The electrical contact comprises an electrically conductive elastomeric material wherein the elastomeric material is compressed between the housing and the printed circuit board connecting the PCB to the sensor, preferably, the electrical contact is being established simultaneous with the mechanical connection.

## Description

### FIELD OF THE INVENTION

The current invention relates to a medical device having a printed circuit board and a sensor. The printed circuit board is mechanically connected to the housing of the medical device and the sensor is connected to the printed circuit board via an electrical contact.

### BACKGROUND OF THE INVENTION

Medical devices are intended to be used for medical treatments. Examples are injection or infusion devices such as injections pen, infusion pumps, patch injectors or bolus injectors. Those examples are used for injecting or infusion a medicament into the patient. Medical devices may be disposable devices or reusable devices or a combination thereof: a part is reused whereas another part, that is preferably releasable connectable to the reusable part, is disposed after use. The medical devices may be part of a medical system where a plurality of devices, including medical devices, are connected to each other preferably in a wireless manner. Medical devices may be add-on devices with a releasable mechanical connection to another device. For example an add-on device comprises the reusable components and the device connected to the add-on device is a disposable device such as an injection pen.

Medical devices have a housing providing mechanical stability to the device enabling a user to hold and manipulate the device. The housing comprises at least an outside housing and preferably comprises internal housing parts providing stability to the internal parts of the medical device. The internal parts may be mechanical components, for example components related to a needle insertion mechanism, a reservoir holder or a fluid delivery mechanism. The internal parts may be electrical or electromechanical components such as a battery, a printed circuit board, wiring, acoustic or visual signaling units, sensors or actuation buttons. The printed circuit board (PCB) may have a control unit, a memory unit, a transmission/receiver unit, signaling units and terminals and wirings for establishing contacts to the units on the PCB or to units separate from the PCB such as, for example the battery, the sensor or the actuation button. Such electrical contacts are crucial for a successful integration and the stability over time and during use are important features for the reliability of the device.

The internal parts, whether mechanical or electrical need to be fixed to the housing or housing parts during assembly of the device. A reliable mechanical fixation over time is important as the devices are often prefabricated having a certain shelf life before use. Additionally, components are purchased from (external) suppliers. The housing and the internal parts are produced separately and each having their respective manufacturing tolerances. Additionally, the dimensions and tolerances may change over time, for example due to physical ageing of the (polymeric) materials.

The assembly of the device involves the marriage of the parts forming the medical device thus establishing the mechanical connections and the electrical contacts between the components. It is preferred to reduce the number of parts during assembly and facilitate the assembly process.

The medical device must be reliable as the accurate delivery of a medicament or monitoring of a patient's condition or monitoring of the functioning of the medical device is crucial. Therefore, the medical device must be robust and able to withstand conditions such as high/low temperatures and must have a sufficient resistance to impact damage without device failure.

In US2005006548 an electronic display module for surface mount construction is disclosed. Plastic components are heat-stake mounted to a printed circuit board substrate for providing the mechanical connection between a plastic (housing) component and the circuit board.

US8885355 discloses a printed circuit board that is heat staked to a housing forming a mechanical connection. The electrical connections for the printed circuit are formed by separate metal clips having arms contacting the PCB.

US2008066306 discloses a mechanical connection between two electrically conductive fabrics using heat staking and the heat staking studs are made from an electrically conductive plastic providing additional paths for the current between the two fabrics.

It is an object of the present invention to overcome the drawbacks of the prior art and provide a medical device with a printed circuit board fixated to a housing by a mechanical connection and a sensor which is connected to the printed circuit board by an electrical contact. The mechanical connection and the electrical contact allow for easy assembly and a stable and reliable mechanical/electrical connection with a high impact tolerance.

This objective is solved by an electrical contact formed by an electrically conductive elastomeric material that is compressed between the housing and the PCB due to a mechanical connection between the housing and the PCB and the electrical contact is formed between the PCB and a sensor. The resilience of the electrically conductive elastomeric material provides for a reliable and long living electrical contact. Furthermore it is an objective to provide an assembly method for the medical device.

### DEFINITIONS

In the claims, the word "comprising" does not exclude other elements or steps, and the indefinite article "a" or "an" does not exclude a plurality. For example "a sensor" or "a terminal" does not exclude the fact that there may be two sensors or terminals that functionally or structurally fulfill the purpose of "a sensor" or "a terminal". The mere fact that certain elements or steps are recited in distinct claims shall not preclude the existence of further meaningful combinations of these elements or steps.

### GENERAL DESCRIPTION OF THE INVENTION

In an aspect of the invention, a medical device is presented comprising a housing or a housing part that is made from a polymeric material. Furthermore, the medical device has a printed circuit board (PCB) fixated to the housing by means of a mechanical connection and a sensor which is located in, or forms part of the housing. An electrical contact is located between the housing and the printed circuit board and formed between the sensor and the printed circuit board. The electrical contact comprises an electrically conductive elastomeric material and the elastomeric material is compressed between the housing and the printed circuit board thereby connecting the PCB to the sensor. Preferably, the electrical contact is being established simultaneous with the mechanical connection.

Preferably, the elastomeric material is sandwiched between the housing and the PCB.

The medical device may be a delivery device for delivery of a medicament such as an infusion pump, an injection device, an injection pen, a patch pump or a patch injector.

Alternatively the medical device may be a device that is connected to another medical device and measures or detects a condition in a patient or a condition or a status of the another medical device. The another medical device can be used fully independent (stand-alone) or used in combination with the medical device whereby the combination provides additional features compared to the stand-alone medical device. The condition measured in a patient may, for example, be a blood glucose value or a temperature. The condition or status measured of the another medical device may be, for example, a value related to the set or expelled dose, or it may be showing "ready to use" or "delivery completed". The medical device may form part of a system comprising several devices and may have a mechanical coupling to the another medical device or may have wireless connections to the other devices in the system. The medical device may have acoustic or optical indicators indicating to the user the patient's condition or the status of the another medical device.

The printed circuit board comprises a control unit for controlling the medical device or the another medical device. The PCB may have a memory unit for storing data. Optionally, the PCB comprises the acoustic or optical indicators such as a vibrating unit, a loudspeaker or LED lights. The PCB unit may have a transmitter/receiver unit for sending data to, or receiving data from, an external device. Communication between the medical device and the another medical device or an external device of the system may be via Bluetooth, low energy Bluetooth, NFC (near field communication), WLAN, Zigbee, Enocean, radio frequency (RF), infra-red (IR) or cellular phone communication.

The printed circuit board may be rigid and/or flexible and may be formed as a single board or comprises a plurality of boards. For example, the PCB may be formed by a two rigid boards connected by a flexible wiring or by one rigid board and a flexible printed circuit board connected thereto.

The sensor may be configured to measure a property such as temperature, speed, geographic position, time, humidity, status of the device (ready-to-use, on-hold, injection completed), malfunctioning of the device (injection not completed, occlusion, jammed button, low battery level, electrical short-cut) or properties related to the data transmission/receiver (data received, transmitted or failure in the data transmission or failure to pair with another device). Optionally, the sensor may detect information related to the production data or expiry date of the device or of the another medical device coupled thereto. In a preferred embodiment, the sensor is a tactile sensor, for example a touch sensitive sensor detecting contact, position, patterns or movements of the fingers of a user.

The sensor may be embedded in the housing and may be located on the outside or on the inside of the housing. The sensor may have an elongated structure having the shape of a slider on a touch screen display. The sensor may be a single sensor or there may be a plurality of sensors that are each located on the inside and/or on the outside of the housing.

The electrical contact is required to transmit signals from the sensor to the PCB and may be formed by electrically conductive contact terminals at the sensor and at the PCB.

An elastomer, preferably a thermoplastic elastomer is used as the compliance enables compensation for dimensional tolerances during manufacturing or use. Additionally, the compliant material may absorb shocks or vibrations acting on the housing and the PCD connected thereto due to the intrinsic elastic properties. The dimensional tolerances may relate to manufacturing tolerances on the housing, the PCB or tolerances due to the assembly itself. The tolerances may appear over time during ageing of the device, particularly due to physical ageing of polymeric materials. Alternatively tolerances between parts may occur due to different thermal expansion coefficients, for example between the PCB and the housing, such that the tolerances may be different at high (above thirty preferably above fifty more preferably above seventy degrees Celsius) or at low temperatures (below zero, preferably below minus ten, more preferably below minus thirty degrees Celsius).

The elastomeric material is compressed between housing and the PCB by the mechanical connection. The compressed elastomeric material may return to a less compressed state to compensate for the dimensional tolerances during manufacturing or ageing of the device, preferably to compensate for dimensional changes or tolerances of the mechanical connection while maintaining the electrical contact.

The compliance and compression of the material thus ensures a reliable mechanical connection between the housing and the PCB and therewith a reliable contact between the sensor and the PCB.

The shock absorbing properties may be beneficial to protect electrical components and circuits on the PCB therewith improving the robustness of the medical device.

The mechanical connection and electrical contact may be simultaneously formed therefore requiring less assembly steps as no electrical contact needs to be formed separate from the mechanical connection

The medical device wherein, the electrical contact comprises a first terminal made from, or located in, or on the surface of the electrically conductive elastomeric material and a second terminal formed as a conductive track at the PCB.

The sensor and the first terminal are preferably unitarily formed.

The first terminal and sensor formed as one part combine two features, the terminal for contacting the PCB and the sensor providing the signals that need to be directed to the PCB via the first terminal. The sensor and the first terminal are electrically connected to each other as they are unitarily formed, combining two features: the terminal and the sensor, thus requiring less parts. Additionally it will be easy to provide a plurality of first terminals.

The medical device wherein the sensor and the first terminal are formed by the electrically conductive elastomeric material.

The sensor may extend from the terminal or may extend between a plurality of first terminals. The sensor may have a flat, sheet like shape and an edge of the flattened shape may be used as the first terminal contacting the second terminal on the PCB. Optionally, the first terminal is within the top or bottom surface of the sheet like sensor. Dimensional tolerances between the PCB and the sensor may be compensated if the PCB is positioned on top of the sensor and the electrical contact between the first and second terminals is established in a plane perpendicular to the flat sensor surface.

In an embodiment, the housing and the sensor are made by two component injection molding of an electrically insulating thermoplast forming the housing and the electrically conductive elastomeric material forming the sensor and the first terminal. The elastomeric material is preferably based on a thermoplastic elastomer.

.Both materials may be injection molded subsequently, alternatively, the sensor is molded first and provided as an inlay in a second molding step, whereby the thermoplastic housing is injection molded around the inlay. Two component injection molding facilitates manufacturing of the sensor and housing and reduces the number of parts that need to be assembled for assembly with the other parts of the medical device.

The medical device wherein the electrically conductive elastomeric material is filled with carbon black forming a percolating network. The elastomeric material may be a natural or synthetic rubber or a thermoplastic elastomer.

The elastomeric material can be injection molded and the elastomer provides for the resiliency and the carbon black provides for the electrical conductivity. The electrical conductivity is required for forming the first terminal and optionally for achieving or contributing to the sensing properties of the sensor. The terminal and the sensor may each have a different carbon black filling grade and/or may each have a different layer thickness. Thicker layers may be more compliant and provide better shock absorbing properties. Optionally, part of the elastomeric material is filled with another filler or with a blend of fillers. For example, carbon black may be combined with carbon nanotubes, metal flakes or silicon carbide to fine tune the electrical and/or sensing properties. Additionally, the carbon black filler or the other fillers may have a monodisperse or a polydisperse particle size distribution.

The medical device wherein the mechanical connection or fixation between the housing and the PCB is selected from heat staking, laser welding, ultrasonic welding, a screw or a snap-fit connection.

The mechanical connection may be realized by heat staking comprising a plastically deformed post which is part of and extends from the housing through a passage in the PCB board.

Preferably, the PCB and the sensor with the first terminal are positioned on top of each other and the electrically conductive elastomeric material at least partially surrounds the post.

The electrically conductive material forming the first terminal may surround the base surface of the post. Alternatively, the post itself is covered by the electrically conductive material or both the post and the base are covered by the electrically conductive material

The electrically conductive elastomeric material forming the first terminal may be directly adjacent and surrounding the base of the post or a gap exists between the post and the first terminal. The elastomeric material surrounding the post may be formed as a continuous layer on the housing or as a discontinuous layer. The layer thickness may be constant or variable. Preferably, the first terminal extends from and is connected to a layer of the elastomeric material covering the housing. The first terminal may be arranged as a continuous or discontinuous circle surrounding the base of the post. The electrically conductive material is connected to the sensor and also forms the first terminal for the electrical contact. The second terminal is preferably located close to the passage in the PCB.

The complementary conductive track forming the second terminal is established at a top or bottom surface of the PCB or within the passage of the PCB.

The second terminal is located close to or surrounds the passage in the PCB. The electrical contact may be established on one side of the PCB, or on both sides of the PCB. Additionally, the electrical contact may be established within the passage and optionally connect the conductive tracks on the top and bottom surfaces. If part of the passage comprises the second terminal, then the post on the housing may also be covered by an electrically conductive surface that is part of the elastomeric material or electrically connected thereto.

If the elastomeric and conductive material forming the first terminal surrounds the base of the post, then the second terminal is preferably located on the top or bottom surface.

In an embodiment, the housing may have a plurality of posts and some of the electrical contacts are established on one side of the PCB whereas other electrical contacts are established at the opposite side of the PCB. Multiple electrical contacts may be used for different purposes, for example for contacting a plurality of sensors each having a different purpose. Alternatively one sensor may be contacted to the PCB via a plurality of contacts using posts passing through passages in the PCB. Multiple contacts may increase the robustness and reduce the vulnerability to impact damage as there are also a plurality of mechanical connections.

An embodiment according to the present invention comprises a housing that is at least partially translucent and the sensor at least partially surrounds the translucent areas.

The translucent area allows for displaying information to the user. The translucent area is part of a visual indicator comprising also a light source located inside the housing adjacent to the translucent area. For example a LED light may be positioned within the housing just below the translucent area. A plurality of optical indicators may be used each having a different color presenting a certain condition to the user (ready to start, injection completed, analysis completed, data sent). The translucent area may be translucent due to a locally reduced wall thickness compared to other part of the housing. In this case, the housing is made from a single polymeric material. Alternatively, a different polymeric material is used for the translucent areas, this may be, for example a different polymeric material such as a polyacrylate or a polypropylene or a blend of the same polymeric material locally having a lower filler content reducing light scattering.

In an embodiment, the translucent areas form the end of a bore at least partially surrounded by the elastomeric and carbon black filled material and wherein the bore is configured to receive a light source.

The shape of the visual indicator once backlighted by the light source may be defined by the sensor surrounding the translucent area. The sensor is preferably made from the carbon black filled elastomeric material which is non-transparent to light. The carbon black filled material is non-translucent and surrounds the ending of the bore which is translucent. Therefore, the transmitted light through the translucent area is presented to the user as having a sharp border or edge to the non-translucent or partially translucent area thereby improving the clarity of the signaling as there is a sharp transition from the translucent to the non-translucent areas.

In a preferred embodiment the medical device comprises a capacitive sensor or a surface touch sensor.

The sensor is preferably present inside the housing and the user approaches the sensor from the outside touching the outside surface of the housing. The wall of the housing and the sensor preferably form a layered structure. The wall of the housing is sandwiched between the sensor and the user touches or slides across the surface. A local change in capacitance or self-capacitance is sensed by the sensor. Preferably, the thickness of the housing is low in the sensor areas to improve detection of a change in capacitance. The layered structure with a non-conductive housing positioned between the electrically conductive layer of the carbon black filled material and the ambient may form the capacitive sensor.

Medical Device may be an add-on device for an injection pen, a patch injector or an auto injector.

Preferably the housing or housing part is adapted to releasably connect to an injection pen.

The releasable connection ensures that the add-on device can be used multiple times, for example in combination with a disposable device such as an injection pen or an auto injector.

A method for assembling the medical device comprising the steps of:
Providing a housing having at least one undeformed/predeformed post for heat staking, or a snap-fit connector or a thread element, and providing the electrically conductive elastomeric material forming the first terminal and the sensor. Providing a printed circuit board with at least one passage and the second terminal, followed by
aligning the printed circuit board with the housing such that the post fits through the passage followed by approaching the PCB towards the housing,
compressing the elastomeric material by the PCB to form the contact between the second terminal on the PCB and the first terminal that is part of the elastomeric conductive material, followed by
fixation of the printed circuit board by heat staking of the post or by snap-fitting the PCB to the housing or screwing the PCB to the housing, thereby keeping the elastomeric material in a compressed state. Alternatively, the PCB is fixed to the housing or to the posts extending from the housing using laser welding, an adhesive or ultrasonic welding.

### BRIEF DESCRIPTION OF THE DRAWINGS

The subject matter of the invention will be explained in more detail in the following text with reference to preferred exemplary embodiments which are illustrated in the attached drawings, in which:
Figure 1: Medical device as an add-on device according to an embodiment of the invention,
Figure 2: Medical device as an add-on device connected to an injection pen,
Figure 3: Housing of the medical device, top view (Fig. 3a) and longitudinal section (Fig. 3b),
Figure 4: Housing of the medical device including a layer of elastomeric material, top view (Fig. 4a) and longitudinal section (Fig. 4b), including details (Fig. 4c and 4d),
Figure 5: Assembly of the housing and the printed circuit board, top view (Fig. 5a) and longitudinal section (Fig. 5b),
Figure 6: Assembly of the housing with a fixated PCB by heat staking of the posts (Fig. 6a), longitudinal section (Fig. 6b) and detail longitudinal section (Fig. 6c),
Figure 7: Detail of the assembled device showing the PCB, a LED and a bore in the housing,
Figure 8: Top and bottom view of the printed circuit board.

### DETAILED DESCRIPTION OF PREFERRED EMBODIMENTS

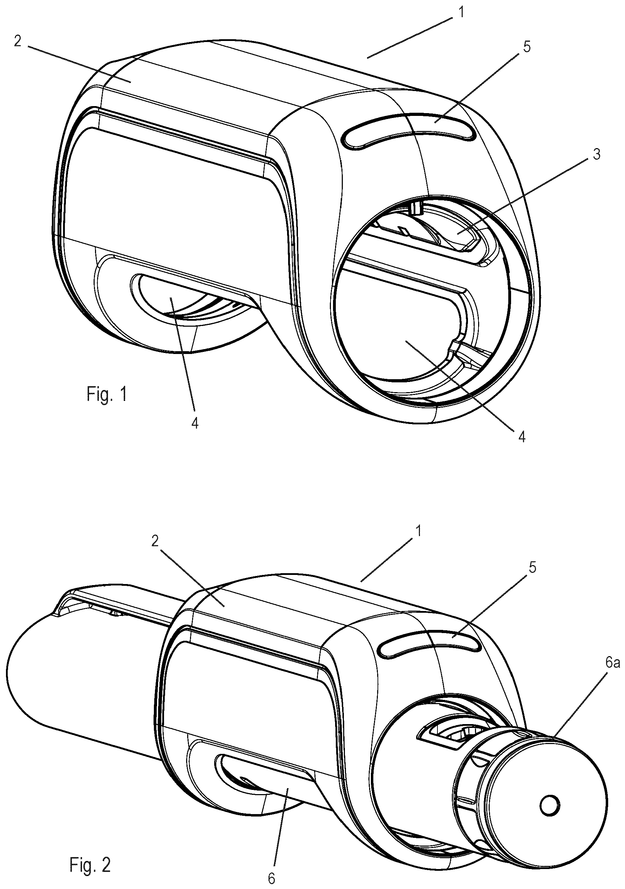

In Figure 1, a medical device 1 is shown as an add-on device for an injection pen 6. The medical device 1 comprises a housing 2 as an outer housing and optionally a second housing part or PCB holding part 3 that may be moveably attached to the housing 2. The moveable part 3 is configured to facilitate insertion of the injection pen 6 via the two passages 4 extending from the housing 2. The housing 2 may further accommodate fixation means for establishing a releasable mechanical connection between the add-on device and the injection pen. The housing 2 may have optical indicators 5.

The assembly of the medical device 1 with another medical device, in this example an injection pen 6, is presented in Figure 2. The injection pen 6 has been inserted through the passages 4 and is mechanically fixed to the add-on device 1, for example by displacing the PCB holder 3 and/or using the fixation means. The PCB holder 3 may be biased with respect to the housing 2 (for example using a spring) such that the add-on device can be releasably connected to the injection pen 6. A not shown actuation button may be pressed to release the connection between the add-on and the pen for inserting a new (disposable) pen. The add-on device is therefore reusable. The injection pen may have a dose dialing mechanism producing audible/tactile clicks during dose setting and these clicks may be detected by a sensor in the add-on device, for example an acoustic or acceleration sensor.

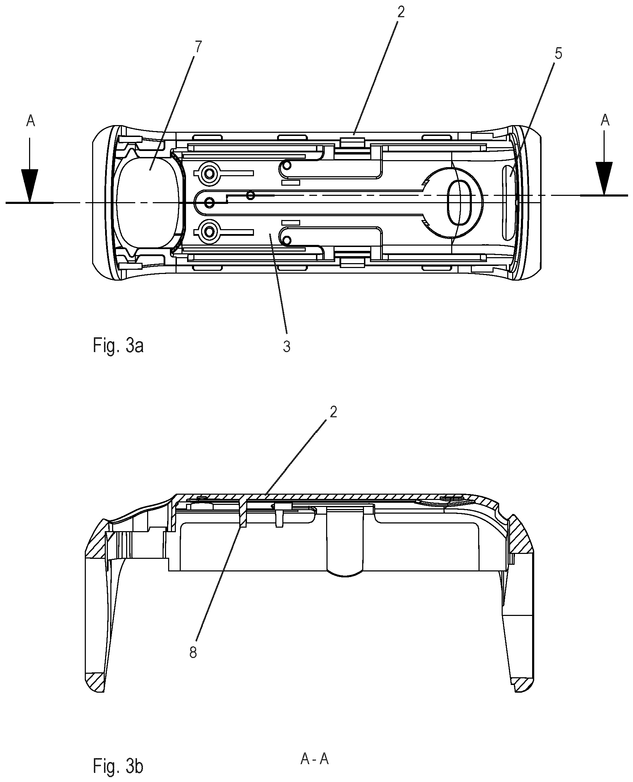

A top view of the housing 2 or printed circuit board holder 3 is shown in Figure 3a. The housing 2 is made from a thermoplastic material for example ABS, a blend of PC/ABS (cycoloy), a polyamide like PA 4-6 or PA 6-6, a polyester like PBT or a polyacetal like POM. The polymeric material is injection molded into the shape of the housing comprising at least posts 8 for fixation of the printed circuit board. Optionally the housing provides the outer shape of the device including the passages for insertion of the injection pen and passages for the actuation button 7 and the light guide 5. The bottom surface may have a textured surface or profile for guiding the flow of the elastomeric material 9 that is injected into the housing 2. Preferably the housing 2 and the elastomeric material are injection molded in a two component injection molding step, first injecting the housing material and subsequently injection molding the elastomeric material 9. Alternatively, the elastomeric material is made in a separate step and provided as an inlay for molding the thermoplastic material surrounding the inlay to form the housing 2.

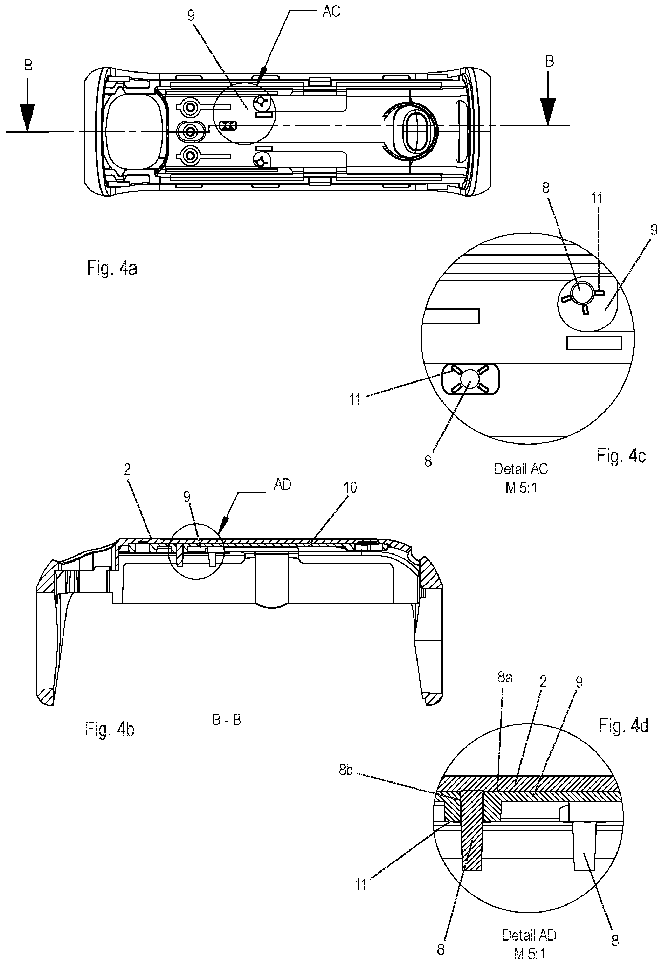

The housing 2 including the elastomeric material 9 is presented in Figures 4a and 4b the elastomeric material 9 covers the bottom surface of the housing 2. A sensor 10 is positioned inside the housing. The sensor is preferably formed from the same elastomeric material 9.

The elastomeric material is preferably an electrically conductive elastomeric material, for example a carbon black filled material. The matrix is made from, for example polypropylene, polyethylene, natural rubber polyisoprene or a thermoplastic elastomer such as a thermoplastic polyurethane. Alternative fillers may be based on metal flakes. Examples are the polypropylene based Pre-Elec TP6735 with a volume resistivity below 20 Ωcm and a Shore-A hardness above 90 (according to ISO868). Alternatively a thermoplastic polyurethane (TPU) can be used such as, for example pre-elec TP17252 with a Shore-A hardness value of 86 and a volume resistivity below 10 Qcm.

A detail of the top view of the surface of the housing is presented in Figure 4c. The base 8a (Figure 4d) for the posts 8 are surrounded by the elastomeric material 9 and a plurality of first terminals 11 extending from the surface layer of the elastomeric material 9. In this embodiment one post is surrounded by 3 first terminals 11 and the other post by 4 first terminals 11 (Figure 4c). The first terminals 11 extend radially outwards from the center of the post 8. Alternatively, the first terminal may be shaped as a continuous ring surrounding the post 8. The detail of the longitudinal section presents the layered structure of the housing 2, the elastomeric material 9 and the first terminal 11 extending from the base 8a of the post 8. In this example, the surface 8b of the post is covered with the elastomeric material as well. Optionally, there may be a gap between the surface of the post 8b and the terminal 11.

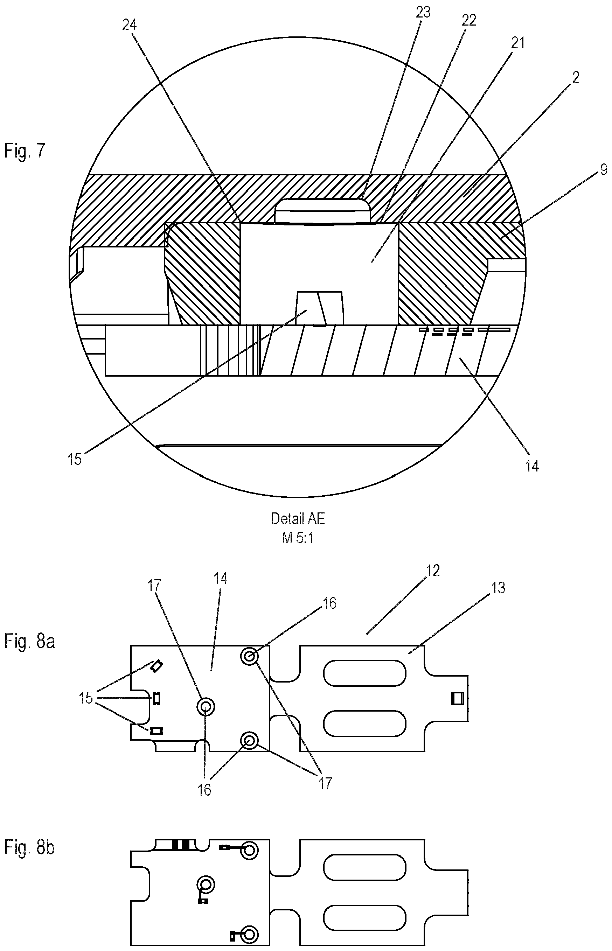

A printed circuit board (PCB) is shown in Figure 8a (top) and 8b (bottom). The PCB 12 comprises a rigid part 14 connected to a flexible circuit board 13. The PCB comprises passages 16 which are surrounded by electrically conductive areas forming the second terminals 17. The electrically conductive areas are formed, for example by a metal layer comprising copper, gold or silver. The electrically conductive areas are either made by additive processes (printing, coating or 3D printing) or by local etching of a continuous conductive layer.

Additionally there are LED lights 15 on the printed circuit board. The PCB may further comprise a control unit and other sensors, for example the aforementioned acoustic or acceleration sensors a touch sensitive sensor.

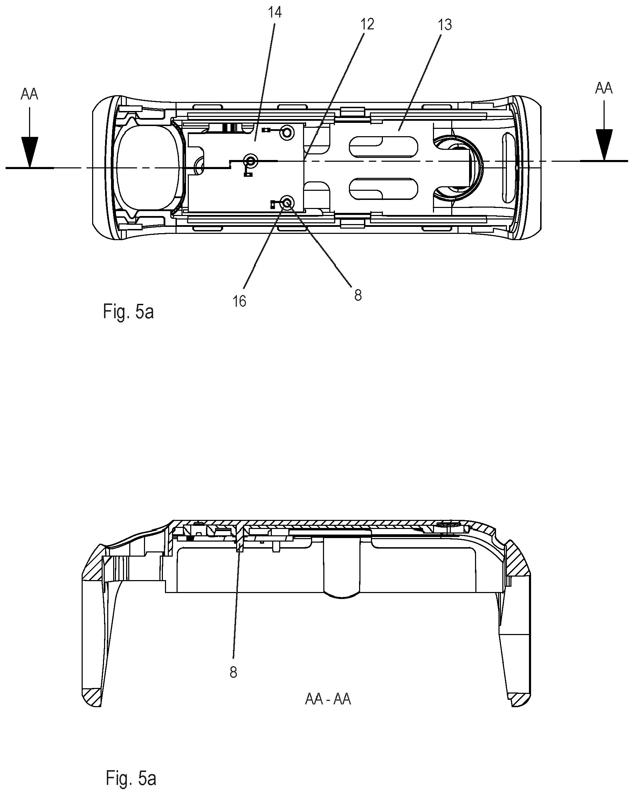

The PCB 12 is positioned into the housing 2 in Figure 5. The posts 8 extend from the housing 2 through the passage 16 in the PCB.

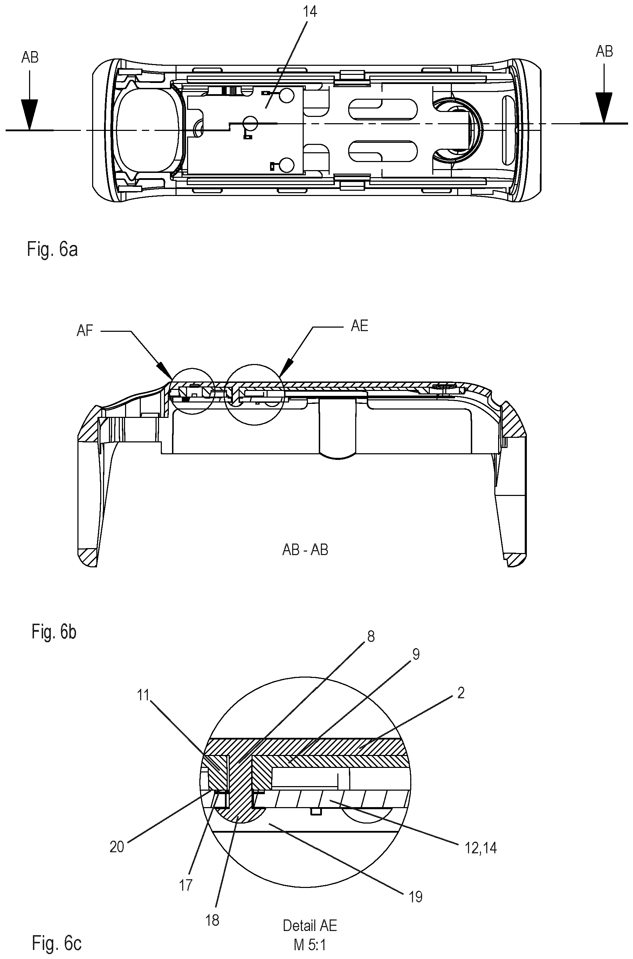

The medical device with the heat staked posts 8 is presented in Figure 6. The end of the posts is plastically deformed to fixate the PCB 12 to the housing 2. Preferably pressure is applied to the PCB before or during heat staking to compress the elastomeric material 9 before or during the fixation. The elastomeric material will provide a resilient force towards the base of the posts 8a and the deformed ending of the posts once the mechanical connection between the housing and the PCB has been established.

During heat staking the electrical contact 20 is formed between the second terminal 17 on the PCB 12,14 and the first terminal 11. In the example presented in Figure 6c, the top surface of the PCB comprises the second terminal 17 and the electrical contact 20 is thus at the top surface of the PCB. Alternatively, the electrical contact 20 is formed at the opposite surface of the PCB. For this option it may be preferred that a part of the post comprises, or is covered with, electrically conductive tracks or that the electrically conductive material surrounding the surface 8d of the post extends through the passage 16 in the PCB 14 prior to heat staking. Compressing the elastomeric material and deformation of the post 8 will provide the electrical contact at the opposite surface of the PCB or, optionally, within the passage 16 connecting the top surface and the bottom surface of the PCB.

As an alternative to heat staking also other fixation means may be used to fixate the PCB to the housing such as a screw type of connection, an adhesive, laser welding or ultrasonic welding.

The resilience provided by the compressed elastomeric and electrically conductive material ensures that manufacturing tolerances in the PCB, the housing and the posts can be compensated for, either initially after manufacturing or after ageing of the medical device. The dimensional changes may be the result of physical ageing (volume reduction over time) and/or due to relaxation of stresses that are frozen in during injection molding and/or due to different thermal expansion coefficients of the PCB, the housing and the elastomeric material, respectively.

The elastomeric and electrically conductive material 9 extends along the surface of the housing to form a sensor 10 (Figure 4b). A multilayered structure is obtained whereby the housing 2 is sandwiched between the sensor 10 and the ambient. Pressing or touching the housing will locally change the capacitance or self-capacitance of the sensor 10 and this sensor signal is sent to, or detected by the PCB via the electrical contact 20. As the conductive material extends along the longitudinal axis of the medical device 2 (along the pen axis in Figure 2), the conductive material 9 may provide a touch sensitive sliding sensor 10.

A detailed view of section AF (Figure 6b) is presented in Figure 7. The LED light source 15 is positioned between the PCB 14 and the housing 2 and is positioned within a bore 21 that is formed by the elastomeric material 9. At the end 22 of the bore 21, the wall of the housing 2 has a thinner section 23 that locally enhances the translucence of the housing. The thinner section 23 may be formed as a lens for focusing the light emitted from the LED. The elastomeric material is, due to the filling with carbon black, non-translucent to the visible light emitted and ensures that no light is diffused outside the area of the bore. As a consequence, the edge of the bore 24 provide for sharp contours surrounding the optical indicator. Using the carbon black filled material thus reduces the need for separate masks. The LED light may be directly transmitted to the bore 21 or via a not shown optical wave guide.

**LIST OF REFERENCE SIGNS**

| | | | |
|---|---|---|---|
| 1 | Medical device, add-on device | 13 | Flexible part PCB |
| 2 | Housing, outer housing | 14 | Rigid part PCB |
| 3 | Housing, PCB holder | 15 | LED |
| 4 | Passage for injection pen | 16 | Passage |
| 5 | Optical indicator / light guide | 17 | Second terminal |
| 6 | Injection pen | 18 | Heat staked ending |
| 6a | Dose dialing mechanism | 19 | Mechanical connection |
| 7 | Passage for actuation button | 20 | Electrical contact |
| 8 | Post | 21 | Bore |
| 8a | Base post | 22 | Ending bore |
| 8b | Surface post | 23 | Thin section housing |
| 9 | Elastomeric material | 24 | Edge bore |
| 10 | Sensor | | |
| 11 | First terminal | | |
| 12 | PCB | | |

## Claims

1. Medical device comprising:
a housing or a housing part made from a polymeric material,
a printed circuit board (PCB) fixated to the housing by means of a mechanical connection,
a sensor located in or forming part of the housing
an electrical contact located between the housing and the printed circuit board and formed between the sensor and the printed circuit board ,
**characterized in that**
the electrical contact comprises an electrically conductive elastomeric material wherein the elastomeric material is compressed between the housing and the printed circuit board connecting the PCB to the sensor, preferably, the electrical contact is being established simultaneous with the mechanical connection.

2. Medical device according to claim 1 wherein, the electrical contact comprises a first terminal made of the electrically conductive elastomeric material and a second terminal formed as a conductive track at the PCB.

3. Medical device according to claim 2 wherein the sensor and the first terminal are unitarily formed.

4. Medical device according to claim 3 wherein the sensor and the first terminal are formed by the electrically conductive elastomeric material.

5. Medical device according to claims 2 to 4 wherein the housing and the sensor are made by 2 component injection molding of an electrically insulating thermoplast forming the housing and the electrically conductive elastomeric material forming the sensor and the first terminal.

6. Medical device according to any of the previous claims wherein the electrically conductive elastomeric material is filled with carbon black forming a percolating network.

7. Medical device according to claims 2 to 6 wherein the mechanical connection includes heat staking, laser welding, ultrasonic welding, a screw or a snap-fit connection.

8. Medical device according to claim 7 wherein the mechanical connection is realized by heat staking comprising a plastically deformed post which is part of and extends from the housing through a passage in the PCB.

9. Medical device according to claim 8 wherein the electrically conductive elastomeric material at least partially surrounds the post.

10. Medical device according to claim 9 wherein the conductive track is established at a top or bottom surface of the PCB or within the passage of the PCB.

11. Medical device according to any of the previous claims wherein the housing is at least partially translucent and wherein the sensor surrounds the translucent areas.

12. Medical device according to claim 11 wherein translucent areas form the end of a bore surrounded by the elastomeric and carbon black filled material and wherein the bore is configured to receive a light source.

13. Medical device according to any of the previous claims wherein the sensor is a capacitive sensor or a surface touch sensor.

14. Medical Device according to any of the previous claims wherein the device is an add-on device for an injection pen or a patch injector.

15. Medical device according to claim 14 wherein the housing or housing part is adapted to releasably connect to an injection pen

16. Method for assembling the medical device according to claims 1 to 15 comprising the steps of:
providing a housing having at least one undeformed/predeformed post for heat staking, and the electrically conductive elastomeric material forming the first terminal and the sensor,
providing a printed circuit board with at least one passage, the second terminal,
aligning the printed circuit board with the housing such that the post fits through the passage followed by approaching the PCD to the housing,
compressing the elastomeric material by the PCB to form a contact between the second terminal and the elastomeric conductive material, followed by
fixation of the printed circuit board by heat staking of the post.
